# EUROPÄISCHE PATENTSCHRIFT

(11) **EP 4 164 554 B1**
(45) Veröffentlichungstag und Bekanntmachung des Hinweises auf die Patenterteilung: **04.03.2026**
(21) Anmeldenummer: 21736975.0
(22) Anmeldetag: 14.06.2021
(51) Int. Cl.: A61F 2/90, A61F 2/852, A61F 2/88, A61F 2/856

(54) **IMPLANTAT ZUR BEHANDLUNG VON ANEURYSMEN**
IMPLANT FOR TREATING ANEURYSMS
IMPLANT POUR TRAITER DES ANÉVRISMES

(30) Priorität: 12.06.2020 DE 102020115605
(43) Veröffentlichungstag der Anmeldung: 19.04.2023
(73) Patentinhaber: Phenox GmbH, 44801 Bochum (DE)
(72) Erfinder: HENKES, Hans, 70192 Stuttgart (DE); MONSTADT, Hermann, 44797 Bochum (DE); HANNES, Ralf, 44137 Dortmund (DE); TRÖSKEN, Volker, 58456 Witten (DE); ROLLA, Stefan, 44869 Bochum (DE)
(74) Vertreter: Schneiders & Behrendt Bochum
(86) Internationale Anmeldenummer: PCT/EP2021/065958
(87) Internationale Veröffentlichungsnummer: WO 2021/250281

(56) Entgegenhaltungen:
- WO-A1-2018/208662
- DE-A1- 102014 110 013
- US-A1- 2019 290 460

## Beschreibung

Die Erfindung betrifft ein Implantat zur Beeinflussung des Blutflusses im Bereich von Aneurysmen, die an Gefäßverzweigungen lokalisiert sind. Derartige Aneurysmen werden auch als Bifurkationsaneurysmen bezeichnet.

Aneurysmen sind zumeist sackartig ausgebildete oder spindelförmige (fusiforme) Erweiterungen der Gefäßwand, die vornehmlich an strukturell geschwächten Stellen der Gefäßwand durch den konstanten Druck des Blutes entstehen. Entsprechend empfindlich und anfällig für Verletzungen sind die Gefäßinnenwände eines Aneurysmas. Die Ruptur eines Aneurysmas führt in der Regel zu erheblichen gesundheitlichen Beeinträchtigungen, im Falle zerebraler Aneurysmen zu neurologischen Ausfällen bis hin zum Tod des Patienten.

Neben chirurgischen Eingriffen, bei denen beispielsweise das Aneurysma mittels eines Clips abgeklemmt wird, sind insbesondere endovaskuläre Methoden zur Behandlung von Aneurysmen bekannt, wobei in erster Linie zwei Ansätze verfolgt werden. Zum einen kann das Aneurysma mit Okklusionsmitteln, insbesondere sog. Coils (Platinspiralen) ausgefüllt werden. Die Coils fördern die Thrombusbildung und sorgen somit für einen Verschluss des Aneurysmas. Zum anderen ist bekannt, den Zugang zum Aneurysma, etwa den Hals eines Beerenaneurysmas, von der Blutgefäßseite aus durch stentähnliche Implantate zu verschließen und vom Blutfluss abzukoppeln. Beide Verfahren dienen dazu, den Blutfluss in das Aneurysma und somit den Druck auf das Aneurysma zu vermindern, im Idealfall zu eliminieren und somit die Gefahr einer Ruptur des Aneurysmas zu reduzieren.

Bei der Verfüllung eines Aneurysmas mit Coils kann es vorkommen, dass die Verfüllung des Aneurysmas unzureichend ist, was die Blutzufuhr in das Aneurysma und somit einen weiterhin bestehenden Druck auf dessen Innenwand zulässt. Die Gefahr einer stetigen Erweiterung des Aneurysmas und schließlich seiner Ruptur besteht weiter, wenn auch in abgeschwächter Form. Darüber hinaus ist die Behandlungsmethode in erster Linie für Aneurysmen mit relativ engem Hals - sogenannten Beerenaneurysmen - geeignet, da ansonsten die Gefahr besteht, dass die Coils aus einem weiten Aneurysmahals in das Blutgefäß ragen und dort thrombogenisieren, was zu Verschlüssen im Gefäß führen kann. Schlimmstenfalls wird ein Coil vollständig aus dem Aneurysma geschwemmt und verschließt Gefäße an anderer Stelle. Um die Coils an ihrem Platz im Aneurysmasack zu halten, wird der Aneurysmahals häufig zusätzlich mit einem speziellen Stent abgedeckt.

Ein anderer intravaskulärer Behandlungsansatz setzt auf sogenannte Flussumlenker (Flow Diverter). Diese Implantate ähneln in ihrer äußeren Erscheinung Stents, die zur Behandlung von Stenosen eingesetzt werden. Da die Aufgabe der Flow Diverter jedoch nicht das Offenhalten eines Gefäßes, sondern der Verschluss des Aneurysmazugangs auf Seiten des Blutgefäßes ist, ist die Maschenweite sehr eng, alternativ sind diese Implantate mit einer Membran überzogen. Nachteilig bei diesen Implantaten ist die Gefahr, dass abgehende Seitenäste in unmittelbarer Nähe des zu behandelnden Aneurysmas mitunter ebenfalls abgedeckt und dadurch mittel- oder langfristig verschlossen werden.

Gefäßverzweigungen, insbesondere Gefäßbifurkationen, sind ein relativ häufiges Phänomen. Das durch eine Arterie im Bereich einer Gabelung auf die Stirnwand aufprallende Blut führt - im Fall einer Schwächung der Gefäßwandung - schnell zu einer Aussackung, die sich dann rasch erweitert. Solche Bifurkationsaneurysmen haben häufig einen weiten Hals, der eine Therapie nur mit Okklusionsspiralen schwierig macht.

Vaskuläre Implantate, die geeignet sind, im Bereich einer Gefäßverzweigung eine "Vergitterung" des Aneurysmaeingangs herbeizuführen, werden beispielsweise in den internationalen Patentanmeldungen WO 2012/113554 A1 oder WO 2014/029835 A1 offenbart. Mit nach dem Setzen des Implantats eingebrachten Okklusionsspiralen kann dann das Aneurysma stillgelegt werden. Möglich ist auch, dass das Implantat selber das Aneurysma in ausreichendem Maße vom Blutstrom abkoppelt. Hierzu kann beispielsweise das Implantat eine Membran aufweisen, die im Bereich des Aneurysmahalses oder vor dem Aneurysmahals platziert wird. Ggf. kann auch allein mit Filamenten, typischerweise Drähten geringen Durchmessers, der Blutstrom zum Aneurysma so weit reduziert werden, dass auf die zusätzliche Einbringung von Okklusionsspiralen oder anderen Okklusionsmitteln in das Aneurysma verzichtet werden kann.

Aus der WO 2018/208662 A1 ist ein Flow Diverter-System zur Behandlung von Bifurkationsaneurysmen bekannt, bei dem zunächst ein stentartiger Flow Diverter mit einer seitlichen Öffnung in das Blutgefäß eingeführt wird. Anschließend wird ein weiterer stentartiger Flow Diverter mit einer seitlichen Öffnung so durch die Öffnung des ersten Flow Diverters geführt, dass sich insgesamt eine Y-Form ergibt, wobei die Verzweigung im Y vor dem Bifurkationsaneurysma liegt. Auf diese Weise soll das Aneurysma vom Blutfluss abgeschnitten werden, der Blutfluss in die abzweigenden Blutgefäße jedoch weitgehend unbeeinflusst bleiben.

Nachteilig macht sich bei diesem Stand der Technik bemerkbar, dass die beiden Teile des endgültigen Flow Diverters nacheinander eingeführt werden müssen. Hierzu ist zunächst die korrekte Platzierung des ersten Flow Diverters notwendig, bevor der zweite Flow Diverter innerhalb des Gefäßsystems sowohl relativ zum Aneurysma als auch relativ zum ersten Flow Diverter korrekt positioniert werden muss. Da bei der Behandlung von Bifurkationsaneurysmen im intrakraniellen Bereich Implantate in der Regel über die Arteria femoralis eingebracht werden, muss der behandelnde Arzt somit eine sehr genaue Steuerung über erhebliche Distanzen vollziehen, wobei sich zusätzlich erschwerend bemerkbar macht, dass die Visualisierung der Implantate, insbesondere auch die präzise Ausrichtung der Öffnungen im Körperinneren schwierig ist.

Ausgehend vom beschriebenen Stand der Technik stellt sich somit die Aufgabe, ein Implantat zur Verfügung zu stellen, mit dessen Hilfe sich Bifurkationsaneurysmen behandeln lassen, das jedoch als Einheit an die Zielposition gebracht werden kann.

Diese Aufgabe wird erfindungsgemäß gelöst durch ein Implantat zur Beeinflussung des Blutflusses im Bereich von Aneurysmen, die an Gefäßverzweigungen lokalisiert sind, wobei das Implantat in einem expandierten Zustand, in dem es im Blutgefäß implantiert wird, und in einem kontrahierten Zustand vorliegt, in dem es durch das Blutgefäß bewegbar ist, wobei das Implantat einen ersten und einen zweiten Geflechtabschnitt aufweist, welche im expandierten Zustand röhrenförmig und deren Wandungen aus einzelnen miteinander verflochtenen Drähten aufgebaut sind, wobei der erste und der zweite Geflechtabschnitt jeweils eine Öffnung in der Wandung aufweisen und zumindest die Größe der Öffnung im zweiten Geflechtabschnitt ausreichend ist für die Hindurchführung des ersten Geflechtabschnitts, wobei die Öffnungen in den Wandungen dadurch erzeugt sind, dass die die Geflechtabschnitte ausbildenden Drähte an den Positionen der Öffnungen radial verdrängt werden, und der erste Geflechtabschnitt so durch die Öffnung in der Wandung des zweiten Geflechtabschnitts geführt ist, dass die Öffnung in der Wandung des ersten Geflechtabschnitts zur Öffnung in der Wandung des zweiten Geflechtabschnitts weist.

Die Zusammenfügung des Implantats erfolgt somit nicht erst innerhalb des Körpers unmittelbar angrenzend an das Aneurysma, sondern vor der Einführung in das Blutgefäßsystem. Entsprechend ist die korrekte Platzierung erheblich einfacher.

Von Bedeutung ist darüber hinaus, dass die Öffnungen in den Geflechtabschnitten dadurch erzeugt werden, dass an den jeweiligen Positionen die den Geflechtabschnitt ausbildenden Drähte seitlich, d. h. radial über den Umfang des Geflechtabschnitts verdrängt werden. Es wird also nicht etwa eine Öffnung durch Herausschneiden von Drähten erzeugt, sondern die Drähte selbst bleiben erhalten, ohne dass eine Unterbrechung in die Drähte eingeführt wird. Die Drähte werden lediglich seitlich verschoben. Dies ist insofern von Bedeutung, als es den verlässlichen Transport des Implantats im komprimierten/kontrahierten Zustand an die Zielposition erlaubt. Der Vorschub von unterbrochenen Drähten hingegen ist häufig nicht ohne Weiteres möglich.

Des Weiteren erlaubt die Ausgestaltung des Implantats mit nicht unterbrochenen Drähten die problemlose Expansion des Implantats an der Zielposition im Blutgefäß. Da erfindungsgemäß die Drähte nicht unterbrochen werden, ist die Expansion unproblematisch.

Schließlich ist ein weiterer Vorteil darin zu sehen, dass sich im Bereich der Öffnungen keine losen Drahtenden befinden, die die angrenzenden Gefäßwände verletzen oder auch in den Kanal des Blutgefäßes weisen und hier für eine Behinderung des Blutstroms sorgen könnten. Das Verhindern einer Verletzung der Gefäßwand ist auch deshalb von besonderer Bedeutung, als die Implantate im Bereich eines Bifurkationsaneurysmas platziert werden, das sich ohnehin durch geschwächte Gefäßwände auszeichnet.

Die Herstellung des erfindungsgemäßen Implantats erfolgt gemäß einer ersten Ausführungsform in der Weise, dass zunächst zwei Geflechtabschnitte mit jeweils einer Öffnung in der Seitenwand erzeugt werden. Anschließend wird der erste Geflechtabschnitt durch die Öffnung im zweiten Geflechtabschnitt geführt und teilweise durch das Innere des zweiten Geflechtabschnitts gezogen. Es erfolgt eine Ausrichtung in der Weise, dass die beiden Öffnungen der Geflechtabschnitte aufeinander zuweisen. Auf diese Weise wird im Übergangsbereich ein Durchlass geschaffen, so dass das vom Stammblutgefäß einströmende Blut problemlos in beide abzweigenden Blutgefäße fließen kann.

Die Zahl der Drähte, die die jeweiligen Geflechtabschnitte ausbilden, beträgt vorteilhafterweise 24 bis 96, weiter bevorzugt 36 bis 64, worunter die Zahl pro Geflechtabschnitt zu verstehen ist. Eine relativ hohe Zahl an Drähten sorgt für eine hohe Oberflächendichte, insbesondere im Bereich vor dem Aneurysmahals, sodass der Bluteinstrom unterbunden wird. Auf der anderen Seite steigt mit der Zahl der Drähte die Steifigkeit des Implantats.

Die Öffnungen in den Wandungen des ersten und/oder des zweiten Geflechtabschnitts sind zweckmäßigerweise ungefähr mittig innerhalb des Geflechtabschnitts angeordnet, wobei sich mittig auf die Längsrichtung bezieht. Es verbleiben somit in beide Richtungen noch jeweils ausreichende Längen des Geflechtabschnitts für die Platzierung in den jeweiligen Armen des Blutgefäßes. Gleichzeitig bewirkt dies, dass ein Arm des Y-förmigen Implantats mindestens teilweise doppellagig ist, d. h. zwei übereinander liegende Geflechtabschnitte aufweist.

Mindestens eine, bevorzugt aber beide Öffnungen weisen eine Größe auf, dass der jeweils andere Geflechtabschnitt durch die Öffnung durchgeführt werden kann. Sinnvoll ist, beide Öffnungen gleich groß auszubilden, um den Blutfluss möglichst gleichmäßig zu gewährleisten, ohne zusätzliche Anströmwiderstände zu schaffen.

Eine weitere zweckmäßige Weiterbildung sieht vor, dass sich im expandierten Zustand in dem Arm des Y-förmigen Implantats mit übereinanderliegenden Geflechtabschnitten diese überlappen. Mit anderen Worten ist in diesem Arm ein Geflechtabschnitt länger ausgebildet als der andere. Die Überlappung liegt vorzugsweise in der Weise vor, dass der innenliegende Geflechtabschnitt über den außenliegenden Geflechtabschnitt hinausragt. Das Ende dieses Arms ist somit nur einlagig, wobei sich im einlagigen Abschnitt auch der innenliegende Geflechtabschnitt an die Gefäßwandung anlegt. Auf diese Weise wird der Anströmwiderstand für das Blut verringert. Die Gefahr von thromboembolischen Effekten wird verringert.

Der erste und der zweite Geflechtabschnitt können in dem Segment, in dem die beiden Geflechtabschnitte gemeinsam verlaufen, am äußeren Ende des Segments miteinander verbunden sein. Hier handelt es sich um eine zweite Ausführungsform des erfindungsgemäßen Implantats, das typischerweise anders hergestellt wird, nämlich indem zunächst eine Geflechtstruktur zur Verfügung gestellt wird, welche im expandierten Zustand röhrenförmig und deren Wandung aus einzelnen miteinander verflochtenen Drähten aufgebaut ist, wobei die Geflechtstruktur einen ersten und einen zweiten Geflechtabschnitt aufweist, die in Längsrichtung aneinandergrenzen und hintereinander angeordnet sind, wobei beide Geflechtabschnitte jeweils eine Öffnung in der Wandung aufweisen und zumindest die Größe der Öffnung im zweiten Geflechtabschnitt ausreichend ist für die Hindurchführung der Geflechtstruktur, wobei die beiden Öffnungen auf entgegengesetzten Seiten der Geflechtstruktur angeordnet sind und der erste Geflechtabschnitt nach innen umgestülpt, durch das Innere des zweiten Geflechtabschnitts geführt und durch die Öffnung im zweiten Geflechtabschnitt von innen nach außen geführt wird, in der Weise, dass die Öffnung in der Wandung des ersten Geflechtabschnitts zur Öffnung in der Wandung des zweiten Geflechtabschnitts weist.

Auch gemäß dieser Ausführungsform wird ein Y-förmiges Implantat zur Verfügung gestellt, das bereits außerhalb des Körpers vollständig vorliegt und eine Abkopplung des Aneurysmas vom Blutfluss erlaubt, während der Fluss durch die Blutgefäße selbst ungehindert bleibt, bei dem man jedoch, anders als bei der ersten Ausführungsform, nicht von zwei getrennten Geflechtabschnitten ausgeht, die zusammengeführt werden, sondern von einer Geflechtstruktur mit zwei Geflechtabschnitten.

Der erste Geflechtabschnitt wird zunächst durch sein eigenes Inneres und sodann zumindest teilweise durch das Innere des zweiten Geflechtabschnitts gezogen, ähnlich wie beim Umstülpen einer Socke. Es ergibt sich ein Arm des Y-förmigen Implantats, der doppellagig vorliegt, d. h. bei dem zwei Geflechtabschnitte übereinander liegen. Im Ergebnis wird ein Implantat erhalten, das dem der ersten Ausführungsform ähnlich ist, allerdings sind die beiden Geflechtabschnitte am Ende des doppellagigen Arms miteinander verbunden, denn das Implantat wird wie beschrieben aus einer einzelnen Geflechtstruktur erzeugt. Die Doppellagigkeit erhöht die Radialkräfte und kann somit die Verankerung des Implantats im Blutgefäß verbessern. Dadurch, dass die beiden Geflechtabschnitte am äußeren Ende des Segments, in dem die beiden Geflechtabschnitte gemeinsam, also doppellagig verlaufen, miteinander verbunden sind, liegen an dieser Stelle keine freien Drahtenden vor, was insofern vorteilhaft ist, als die Gefahr von Verletzungen der Gefäßwandung reduziert wird. Zudem wird verhindert, dass Drahtenden in das Gefäßlumen vorstehen (sog. Fischmaul-Effekt) und den Blutstrom behindern.

Bei der Standardplatzierung des Implantats befindet sich der doppellagige Arm des Y-förmigen Implantats im Stammblutgefäß, von dem aus die beiden abzweigenden Blutgefäße ausgehen.

Die Geflechtstruktur, die bei der zweiten Ausführungsform zwei in Längsrichtung hintereinander liegende Geflechtabschnitte aufweist, kann z. B. aus 32 bis 48 Drähten aufgebaut sein, wobei gegenüber der ersten Ausführungsform zu berücksichtigen ist, dass sich die Drähte zwischen den Geflechtabschnitten fortsetzen, während bei der ersten Ausführungsform jeder Geflechtabschnitt für sich aus einer bestimmten Zahl an Drähten aufgebaut ist.

Wie bei der ersten Ausführungsform sind die Öffnungen in den Wandungen des ersten und/oder des zweiten Geflechtabschnitts jeweils zweckmäßigerweise ungefähr mittig innerhalb des Geflechtabschnitts angeordnet, wobei sich mittig auf die Längsrichtung bezieht. Es verbleiben somit in beide Richtungen noch jeweils ausreichende Längen des Geflechtabschnitts für die Platzierung in den jeweiligen Armen des Blutgefäßes. Auch bei dieser Ausführungsform weisen mindestens eine, bevorzugt aber beide Öffnungen eine Größe auf, dass der jeweils andere Geflechtabschnitt durch die Öffnung durchgeführt werden kann.

Bei beiden Ausführungsformen ist von den 3 Armen des Y-förmigen Implantats somit einer mindestens teilweise doppellagig, die anderen beiden sind einlagig. Der doppellagige Arm erzeugt im Zweifel höhere Radialkrafte und ermöglicht somit eine sichere Fixierung des Implantats im Blutgefäßsystem, insbesondere im Stammblutgefäß, von dem die beiden anderen Blutgefäße abzweigen.

Dort, wo sich die Verzweigung zwischen erstem und zweitem Geflechtabschnitt nach Zusammenfügung des Implantats befindet, grenzt normalerweise, also bei der Standardplatzierung, das zu behandelnde Aneurysma an. Da unmittelbar angrenzend an den Aneurysmahals die Geflechtabschnitte anliegen, wird dieses weitgehend vom Blutfluss abgeschnitten. Durch die sich gegenüberliegenden bzw. aufeinander zuweisenden Öffnungen in den Geflechtabschnitten wird jedoch der Blutstrom in die eigentlichen Blutgefäße sichergestellt.

Möglich ist auch eine Platzierung in der Weise, dass eine andere Verzweigungsstelle des Y-förmigen Implantats vor dem Aneurysmahals platziert wird. In diesem Fall verläuft nur ein Teil des ersten oder zweiten Geflechtabschnitts durch das Stammblutgefäß, während der Bereich, in dem die beiden Geflechtabschnitte übereinander liegen, in eines der beiden abzweigenden Blutgefäße eingebracht ist. In dem anderen abzweigenden Blutgefäß befindet sich schließlich der dritte Arm des Y-förmigen Implantats, der aus einer einfachen Schicht des anderen Geflechtabschnitts aufgebaut ist. Eine solche Platzierung kann insofern Vorteile aufweisen, als die Abdeckungsrate unmittelbar vor dem Aneurysmahals unter Umständen größer ist, weil an dieser Stelle eine höhere Geflechtdichte vorliegt.

Um die Fixierung der beiden Geflechtabschnitte aneinander zu verbessern, können diese im Bereich ihrer Öffnungen aneinander befestigt sein. Dies kann insbesondere durch Vernähen, insbesondere mithilfe von Drähten, durch Bildung von Schlaufen um die aneinandergrenzenden Drähte der beiden Geflechtabschnitte, Kleben o. ä. erfolgen. Auf diese Weise wird ein Implantat geschaffen, bei dem die beiden Geflechtabschnitte eine feste Verbindung miteinander aufweisen.

Das erfindungsgemäße Implantat weist eine geflochtene Struktur auf, d. h. das Implantat besteht aus Drähten, die miteinander verflochten werden, indem sie über- und untereinander hergeführt werden. Eine solche Struktur ist besonders geeignet, nach Freisetzung im Blutgefäß zu expandieren und sich an die Gefäßwandungen anzupassen.

Das Implantat koppelt das Bifurkationsaneurysma weitgehend oder vollständig vom Blutfluss ab, da zumindest ein Teil der Drähte vor dem Aneurysmahals zu liegen kommt. Der Blutfluss durch das Stammblutgefäß in die abzweigenden Blutgefäße wird hingegen praktisch nicht beeinflusst. In der Folge verödet das Aneurysma, indem sich mangels Blutbewegung im Aneurysma dort ein Thrombus ausbildet, der das Aneurysma verschließt.

Unter einer röhrenförmigen Geflechtstruktur bzw. einem röhrenförmigen Geflechtabschnitt wird eine Struktur verstanden, bei der die Drähte die Wandung der Röhre bilden. Es handelt sich somit um ein Rundgeflecht. Vorzugsweise haben die Geflechtabschnitte/die Geflechtstruktur vom proximalen oder distalen Ende aus betrachtet einen kreisförmigen Querschnitt. Grundsätzlich sind jedoch auch Abweichungen von der Kreisform möglich, beispielsweise ein ovaler Querschnitt.

Das Geflecht kann im Prinzip auf jede bekannte Art und Weise geflochten werden. Es liegt ein- und/oder mehrflechtig vor. Eine enge Flechtigkeit führt insbesondere bei einem dichten Flechtwerk zu einer hohen Beanspruchung der einzelnen Drähte. Insoweit ist die mehrflechtige Ausführung geeignet, Spannung aus dem Flechtwerk zu nehmen, wobei aber eine zu hohe Flechtigkeit zu einem schlechten Verbund im Geflecht führt. Die Flechtigkeit gibt an, an wie vielen sich mit dem Draht kreuzenden Drähten ein bestimmter Draht auf derselben Seite vorbeigeführt wird, bevor er die Seite wechselt, um anschließend an einer entsprechenden Zahl von kreuzenden Drähten auf der anderen Seite vorbeigeführt zu werden. Bei einer zweiflechtigen Ausführung z. B. wird ein Draht nacheinander über zwei den Draht kreuzende Drähte, dann nacheinander unterhalb von zwei kreuzenden Drähten geführt. In der einflechtigen Struktur liegen die Drähte alternierend übereinander und untereinander.

Die Drähte können insbesondere auch mehrfach gefacht sein. Die Fachung gibt die Anzahl der zusammengefassten, parallellaufenden Einzeldrähte an. Möglich ist eine Einfach- oder Mehrfachfachung, bei der jeweils ein oder mehrere Einzeldrähte parallel laufen. Da bei der Fertigung des Geflechts die Drähte in der Regel von Spulen zugeführt werden, bedeutet dies, dass von der entsprechenden Spule ein bzw. mehrere Einzeldrähte gleichzeitig dem Dorn, auf dem das Geflecht gefertigt wird, zugeführt werden. Jeder Draht kann aus einem einzelnen Draht oder auch aus einer Litze von mehreren zusammengefassten und vorzugsweise miteinander verdrillten Einzeldrähten bestehen.

Eine Fachung von zwei oder eine noch höhere Fachung bringt eine höhere Oberflächendichte des Geflechts mit sich, bei gleichzeitiger Verringerung der Längenausdehnung während der Komprimierung des Geflechts. Diese höhere Oberflächendichte geht allerdings auf Kosten der Flexibilität, auch durch Erhöhung der Reibung und Spannung. Dem kann durch eine Erhöhung der Flechtigkeit entgegengewirkt werden, d. h. eine zwei- oder höherflechtige Struktur bringt eine Erhöhung der Flexibilität mit sich.

Als Drähte können unterschiedliche Formen von Filamenten verwendet werden. Diese können einen runden, ovalen oder auch eckigen Querschnitt, insbesondere rechteckigen, quadratischen oder trapezförmigen Querschnitt aufweisen, wobei im Falle eines eckigen Querschnitts die Kanten abgerundet sein können. Möglich ist auch die Verwendung von flachen Drähten in Form dünner Streifen. Die einzelnen Drähte können sich auch aus mehreren, miteinander verwundenen oder auch parallel verlaufenden Einzelfilamenten zusammensetzen. Die Drähte können massiv oder auch innen hohl sein. Zusätzlich können die Drähte einer Elektropolitur unterzogen werden, um sie glatter und abgerundeter und damit weniger traumatisch werden zu lassen. Dadurch sinkt zudem die Gefahr der Anhaftung von Keimen oder sonstigen Verunreinigungen.

Bevorzugt werden die Drähte aus Metall gefertigt, grundsätzlich ist jedoch auch die Verwendung von Drähten aus anderen Materialien, beispielsweise Kunststoffen bzw. Polymermaterialien denkbar. Auch solche Filamente werden erfindungsgemäß als Drähte verstanden.

Um zu gewährleisten, dass das Implantat nach Freisetzung im Blutgefäß, beispielsweise von oder aus einem Katheter heraus, automatisch expandiert und sich an die Innenwandungen der Blutgefäße anpasst, ist es bevorzugt, die Drähte mindestens teilweise aus einem Material mit Formgedächtniseigenschaften zu fertigen. Besonders bevorzugt sind in diesem Zusammenhang Nickel-Titan-Legierungen, beispielsweise Nitinol, oder auch ternäre Nickel-Titan-Chrom-Legierungen oder Nickel-Titan-Kupfer-Legierungen. Ebenso sind jedoch auch andere Formgedächtnismaterialien, beispielsweise andere Legierungen oder auch Formgedächtnispolymere denkbar. Materialien mit Formgedächtniseigenschaften erlauben es, einem Implantat eine Sekundärstruktur aufzuprägen, die es automatisch bestrebt ist einzunehmen, sobald es nicht mehr an der Expansion behindert wird.

Möglich ist auch die Verwendung von sog. DFT^{®}(Drawn Filled Tubing)-Drähten, d. h. Drähten, bei denen der Kern des Drahtes aus einem anderen Material besteht als der den Kern umgebende Mantel. Insbesondere bietet es sich an, Drähte mit einem Kern aus einem röntgensichtbaren Material und einem Mantel aus einem Material mit Formgedächtniseigenschaften zu verwenden. Das röntgensichtbare Material kann beispielsweise Platin, eine Platin-Iridium-Legierung oder Tantal sein, das Material mit Formgedächtniseigenschaften ist wie bereits erwähnt bevorzugt eine Nickel-Titan-Legierung. Derartige DFT^{®}-Drähte werden beispielsweise von Fort Wayne Metals angeboten.

Entsprechende Drähte vereinigen die vorteilhaften Eigenschaften zweier Materialien. Der Mantel mit Formgedächtniseigenschaften sorgt dafür, dass das Implantat expandieren und sich an die Gefäßwandungen anpassen kann, das röntgensichtbare Material dafür, dass das Implantat im Röntgenbild sichtbar ist und damit vom behandelnden Arzt zu beobachten und entsprechend positionierbar ist.

Die distalen und proximalen Enden der Drähte sind vorzugsweise so ausgestaltet, dass eine Verletzung der Gefäßwände ausgeschlossen ist. Beispielsweise können Drähte an ihren Enden abgerundet und damit atraumatisch sein. Entsprechende Umformungen können durch Umschmelzen mit Hilfe eines Lasers vorgenommen werden. Möglich ist es auch, jeweils einen oder mehrere Drähte an den jeweiligen Enden zusammenzuführen und dabei einen möglichst atraumatisch geformten Abschluss zu schaffen. Insbesondere ist es zweckmäßig, spitze Drahtenden zu vermeiden.

Vorteilhafte Abdeckungsraten des Implantats, insbesondere auch an der Verzweigungsstelle, an der implantiert der Aneurysmahals abgedeckt wird, betragen für das Implantat im expandierten Zustand 45 bis 75 %, vorzugsweise 35 bis 65 %.

Unter expandiertem Zustand wird, soweit sich aus dem Zusammenhang nichts anderes ergibt, erfindungsgemäß ein Zustand verstanden, den das Implantat einnimmt, wenn es keinen äußeren Beschränkungen unterworfen ist. Je nach Durchmesser der Blutgefäße, in denen das Implantat implantiert wird, kann der expandierte Zustand im Blutgefäßsystem vom expandierten Zustand ohne äußere Beschränkungen abweichen, weil das Implantat womöglich nicht seinen vollständig expandierten Zustand annehmen kann. Im vollständig expandierten Zustand weisen die Geflechtabschnitte vorteilhafterweise einen Außendurchmesser zwischen 1,5 mm und 7 mm auf, der an den jeweiligen Einsatzort im Blutgefäßsystem angepasst sein kann. Die Gesamtlänge des Implantats im expandierten Zustand liegt in der Regel zwischen 5 mm und 100 mm, insbesondere zwischen 10 und 50 mm, wenn das Implantat so gelegt wird, dass die beiden einlagigen Arme des Y-förmigen Implantats parallel und in der gleichen Richtung verlaufen wie der doppellagige Arm. Die das Implantat ausbildenden Drähte können z. B. einen Durchmesser bzw. eine Stärke zwischen 20 und 60 µm aufweisen.

Auf der anderen Seite kann das Implantat auch in einem kontrahierten oder komprimierten Zustand vorliegen, wobei die Begriffe im Rahmen dieser Erfindung synonym in dem Sinne gebraucht werden, dass das Implantat bzw. ein Geflechtabschnitt/die Geflechtstruktur im kontrahierten/komprimierten Zustand eine deutlich kleinere radiale Ausdehnung aufweist als im expandierten Zustand. Ein kontrahierter/komprimierter Zustand wird z. B. angenommen, wenn das Implantat durch einen Katheter an die Zielposition gebracht wird. Möglich ist auch die Aufbringung des Implantats außen auf einem Katheter, einem Schlauch o. ä., wobei in diesem Fall das Implantat ebenfalls in einem gegenüber dem expandierten Zustand weniger radial ausgedehnten Zustand gehalten wird.

Die Begriffe "proximal" und "distal" sind so zu verstehen, dass sie beim Einsetzen des Implantats zum behandelnden Arzt weisende Teile des Implantats bezeichnen (proximal) bzw. vom behandelnden Arzt weg weisende Teile (distal). Das Implantat wird somit typischerweise durch oder mit Hilfe eines Katheters in distaler Richtung vorgeschoben. Der Begriff "axial" bezieht sich auf die von proximal nach distal verlaufende Längsachse des Implantats, der Begriff "radial" auf hierzu senkrechte Ebenen.

Um den Verschluss des Aneurysmas weiter zu verbessern, können neben der Abkopplung des Aneurysmas vom Blutfluss durch das Implantat zusätzlich Okklusionsmittel in das Aneurysma eingeführt werden, beispielsweise Coils, wie sie aus dem Stand der Technik bekannt sind. Ebenfalls möglich ist die Einbringung zähflüssiger Embolisate wie Onyx.

Das erfindungsgemäße Implantat weist in der Regel röntgensichtbare/röntgendichte Markerelemente auf, die die Visualisierung und Platzierung am Implantationsort erleichtern. Solche Markerelemente können z. B. in Form von Drahtwicklungen, als Manschetten und als geschlitzte Rohrabschnitte vorliegen, die am Implantat festgelegt werden. Für die Markerelemente kommen als Materialien insbesondere Platin und Platinlegierungen in Frage, beispielsweise eine Legierung aus Platin und Iridium, wie sie vielfach im Stand der Technik für Markerzwecke und als Material für Okklusionscoils eingesetzt wird. Andere verwendbare röntgensichtbare Metalle sind Tantal, Gold und Wolfram. Eine weitere Möglichkeit besteht in der oben bereits erwähnten Füllung der Drähte mit einem röntgensichtbaren Material. Möglich ist auch, das Implantat, insbesondere die Drähte, mit einer Beschichtung aus einem röntgendichten Material zu versehen, beispielsweise mit einer Goldbeschichtung. Diese kann z. B. eine Stärke von 1 bis 6 µm aufweisen. Die Beschichtung mit einem röntgendichten Material muss nicht das gesamte Implantat umfassen. Auch beim Vorsehen einer röntgendichten Beschichtung kann es allerdings sinnvoll sein, zusätzlich einen oder mehrere röntgendichte Marker am Implantat anzubringen, insbesondere am distalen Ende des Implantats.

Das Implantat kann auch über die Geflechtabschnitte zumindest teilweise abdeckende Membranen verfügen, wobei sowohl eine Membran verwendet werden kann, die sich über größere Bereiche der Geflechtabschnitte erstreckt, als auch eine Mehrzahl von kleinen Membranen. Eine solche Membran ist insbesondere am Aneurysmahals sinnvoll, um das Aneurysma vom Blutstrom abzuschneiden, d. h. an der Verzweigungsstelle in Richtung distal.

Unter einer Abdeckung durch eine Membran wird jede Art von Abdeckung verstanden, d. h. die Membran kann auf der Außenseite der Geflechtabschnitte aufgebracht, an der Innenseite der Geflechtabschnitte angebracht sein oder die Drähte der Geflechtabschnitte sind in die Membran eingebettet.

Wenn eine oder mehrere Membranen verwendet werden, ist es auch möglich, röntgensichtbare Substanzen in die Membranen einzubringen. Hierbei kann es sich um röntgensichtbare Partikel handeln, wie sie üblicherweise in der Röntgentechnik als Kontrastmittel eingesetzt werden. Solche röntgendichten Substanzen sind beispielsweise Schwermetallsalze wie Bariumsulfat oder lodverbindungen. Die Röntgensichtbarkeit der Membran ist bei der Einbringung und Lokalisierung des Implantats hilfreich und kann zusätzlich oder anstelle von Markerelementen Verwendung finden.

Membranen können auch so beschaffen sein, dass sie eine antithrombogene oder die Endothelbildung fördernde Wirkung haben. Eine solche Wirkung ist insbesondere dort wünschenswert, wo das Implantat an normale Gefäßwandungen angrenzt, weil der Blutfluss durch die Gefäße nicht beeinträchtigt und darüber hinaus eine gute Verankerung des Implantats im Blutgefäßsystem erreicht werden soll. Die entsprechenden Eigenschaften können die Membranen durch entsprechende Wahl des Materials von sich aus aufweisen, sie können jedoch auch mit diese Wirkungen hervorrufenden Beschichtungen versehen sein.

Als Membran wird erfindungsgemäß eine dünne Struktur mit flächiger Ausdehnung verstanden, unabhängig davon, ob diese für Flüssigkeiten durchlässig, undurchlässig oder teildurchlässig ist. Zur Erfüllung des Zwecks der Behandlung eines Aneurysmas sind allerdings Membranen bevorzugt, die für Flüssigkeiten wie Blut vollständig oder zumindest weitgehend undurchlässig sind. Darüber hinaus kann eine Membran insbesondere im Bereich des Aneurysmahalses auch mit Poren versehen sein, durch die Okklusionsmittel in das Aneurysma eingebracht werden können. Eine weitere Möglichkeit besteht darin, die Membran so auszubilden, dass sie mit einem Mikrokatheter zur Einführung von Okklusionsmitteln oder auch mit den Okklusionsmitteln selbst durchstoßen werden kann.

Die Membranen können aus Polymerfasern oder -folien aufgebaut sein. Vorzugsweise werden die Membranen durch Elektrospinnen erzeugt. Dabei werden normalerweise die Drähte in die Membran eingebettet. Dies kann dadurch erreicht werden, dass die Drähte mit Fasern umsponnen bzw. umflochten werden.

Beim Elektrospinnen werden Fibrillen bzw. Fasern aus einer Polymerlösung mit Hilfe von elektrischem Strom auf einem Substrat abgeschieden. Bei der Abscheidung verkleben die Fibrillen zu einem Vlies. In der Regel haben die Fibrillen einen Durchmesser von 100 bis 3.000 nm. Durch Elektrospinnen gewonnene Membranen sind sehr gleichmäßig ausgebildet. Die Membran ist zäh und mechanisch belastbar und kann mechanisch durchstoßen werden, ohne dass die Öffnung zum Ansatzpunkt für weitergehende Risse wird. Die Dicke der Fibrillen wie auch der Grad der Porosität kann durch Auswahl der Verfahrensparameter gesteuert werden. Im Zusammenhang mit der Schaffung der Membran und den dafür geeigneten Materialien wird insbesondere auf die WO 2008/049386 A1, die DE 28 06 030 A1 und die darin genannte Literatur hingewiesen.

Statt durch Elektrospinnen können die Membranen auch über einen Tauch- oder Sprühprozess wie Spraycoaten hergestellt werden. Wichtig ist hinsichtlich des Materials der Membranen, dass diese durch die mechanische Beanspruchung beim Einbringen in das Blutgefäßsystem nicht beschädigt werden. Die Membranen sollten daher eine ausreichende Elastizität aufweisen.

Die Membranen können aus einem Polymermaterial wie Polytetrafluorethylen, Polyester, Polyamiden, Polyurethanen, Polyolefinen oder Polysulfonen hergestellt sein. Besonders bevorzugt sind Polycarbonaturethane (PCU). Wünschenswert ist insbesondere eine integrale Verbindung der Membranen mit den Drähten. Eine solche integrale Verbindung kann durch kovalente Bindungen zwischen den Membranen und den Drähten erreicht werden. Die Ausbildung von kovalenten Bindungen wird durch eine Silanisierung der Drähte gefördert, d. h. durch eine chemische Anbindung von Siliciumverbindungen, insbesondere Silanverbindungen an zumindest Teilen der Oberfläche der Drähte. Silicium- und Silanverbindungen binden an Oberflächen beispielsweise an Hydroxy- und Carboxy-Gruppen. Neben der Silanisierung sind grundsätzlich auch andere Methoden der Haftvermittlung zwischen Drähten und Membranen denkbar.

Unter einer Silanverbindung sind in diesem Zusammenhang all jene Verbindungen zu verstehen, die der allgemeinen Formel RₘSiXₙ folgen (m, n = 0-4) wobei R für organische Reste, insbesondere Alkyl-, Alkenyl- oder Arylgruppen, und X für hydrolysierbare Gruppen, insbesondere OR, OH oder Halogen mit R = Alkyl, Alkenyl oder Aryl steht. Insbesondere kann das Silan die allgemeine Formel RSiX₃ haben.

Darüber hinaus gehören entsprechende Verbindungen mit mehreren Siliciumatomen zu den Silanverbindungen. Insbesondere Silan-Derivate in Form von siciliumorganischen Verbindungen werden in diesem Zusammenhang als Silanverbindungen aufgefasst.

Wie oben bereits erwähnt, können zusätzliche die Endothelbildung fördernde Stoffe In die Membranen eingebettet oder auf die Membranen aufgebracht sein. Da Aneurysmen auf degenerativen Gefäßwanderkrankungen beruhen, kann die Förderung der Endothelbildung und die Behebung von Funktionsstörungen des Endothels positive Wirkungen haben. Dies gilt insbesondere für den Bereich, wo das Aneurysma Kontakt zum Blutstrom im eigentlichen Blutgefäß (Stammblutgefäß) hat. Vorzugsweise sind die die Endothelbildung fördernden Stoffe auf der Außenseite der Membran aufgebracht, wobei unter Außenseite die im implantierten Zustand der Gefäßwandung zugewandte Seite einer Membran und unter Innenseite die der Gefäßwandung abgewandte Seite einer Membran verstanden wird. Hyaluronsäure, Statine (3-Hydroxy-3-Methylglutaryl-Coenzym-A-Reduktase-Inhibitoren) und andere Polymere können die Besiedlung mit Endothelzellen fördern. Als Polymere eignen sich insbesondere Polysaccharide, besonders Glykosaminoglykane, die in der Lage sind, die Glykokalyx zu imitieren. Ein anderes verwendbares Material ist POSS-PCU (polyhedral oligomeric silsesquioxane poly(carbonate-urea) urethane). Es handelt sich um ein Nanokomposit, das u. a. als Gerüst für künstliche Organe sowie als Coating für medizinische Vorrichtungen beschrieben wurde (Tan et al., Crit Rev. Biomed Eng. 2013; 41(6): 495-513). Möglich ist auch die Verwendung von POSS-PCL (polyhedral oligomeric silsesquioxane poly(caprolactone-urea) urethane). Sowohl für POSS-PCU als auch für POSS-PCL gilt, dass insbesondere auch funktionalisierte Derivate dieser Nanokomposite verwendet werden können. Dies gilt insbesondere für solche Derivate, die sich durch Verknüpfung mit Polyacrylsäure (Poly-AA) erhalten lassen. POSS-PCU- bzw. POSS-PCL-Nanokompositpolymere sind für die unmittelbare Immobilisierung auf der Oberfläche eines Implantats nur schlecht geeignet ist, weshalb sich als vorteilhaft herausgestellt hat, Polymere wie Polyacrylsäure (Poly-AA) mit dem Nanokomposit zu verbinden. Dies kann beispielsweise durch Plasmapolymerisation von Acrylsäure geschehen. Eine auf diese Weise erhaltene Poly-AA-g-POSS-PCU-Oberfläche fördert die Bindung von Kollagen (insbesondere Kollagen Typ 1) und somit die Endothelbildung (vgl. Solouk et al., Mater Sci Eng C Mater Biol Appl. 2015; 46: 400-408). Allgemein können biofunktionelle oder bioaktive Beschichtungen auf der Membran vorhanden sein.

Das erfindungsgemäße Implantat eignet sich insbesondere zur Behandlung von intrakraniellen Bifurkationsaneurysmen, denkbar ist jedoch auch die Verwendung für andere Arten von Aneurysmen, beispielsweise Aortenaneurysmen oder peripheren Aneurysmen, wobei die Dimensionen des Implantats entsprechend anzupassen sind.

Um das Implantat vor dem Bifurkationsaneurysma zu platzieren kann ein Einführsystem verwendet werden, wie es in der WO 2018/134097 A1 beschrieben wird. Als Weiterentwicklung hierzu dient ein Einführsystem, das eine Hülse mit zwei distalen Hülsenarmen aufweist, die zur Aufnahme jeweils eines distalen Implantatarms konfiguriert und miteinander verbunden sind, wobei die Hülse in distaler Richtung eine kontinuierliche Öffnungszone aufweist und die Hülse einen proximalen Hülsenarm zur Aufnahme des proximalen Implantatarms aufweist, wobei sich die Hülse über den proximalen Hülsenarm in proximaler Richtung zurückziehen lässt, sodass sich die Öffnungszone öffnet und die distalen Implantatarme jeweils durch die Öffnungszone treten und in den abzweigenden Blutgefäßen freigesetzt werden, wobei die Öffnungszone so beschaffen ist, dass sich die Hülse bei der Freisetzung des Implantats sequentiell von den distalen Enden der distalen Hülsenarme ausgehend in Richtung proximal öffnet. Gegenüber dem in der WO 2018/134097 A1 beschriebenen Einführsystem unterscheidet sich ein solches Einführsystem dadurch, dass das Implantat nach und nach von distal nach proximal freigesetzt wird und sich entsprechend aufweiten und an die Gefäßwandung anlehnen kann, was als vorteilhaft angesehen wird.

Die Abschnitte des Implantats, die in den abzweigenden Blutgefäßen platziert werden, werden in diesem Zusammenhang als distale Implantatarme bezeichnet, der Abschnitt zur Platzierung im Stammblutgefäß als proximaler Implantatarm.

Die Öffnungszone kann hierzu in unterschiedlicher Weise ausgestaltet sein. Insbesondere kann die Öffnungszone von vornherein einen durchgehenden Schlitz aufweisen, der in distale Richtung weist, wobei sich aber die Kanten des Schlitzes zumindest teilweise überlappen. Diese Überlappung sollte von den beiden distalen Enden der Hülsenarme in Richtung proximal zunehmen. Mit anderen Worten überlappen die jeweiligen Kanten der Schlitze an den distalen Enden der Hülsenarme nicht oder allenfalls geringfügig, dort, wo sich die Hülsenarme treffen und miteinander verbunden sind, hingegen überlappen sich die beiden Kanten des Schlitzes deutlich. Entsprechend wird zur Freisetzung der distalen Implantatarme an den distalen Enden der Hülsenarme am wenigsten Kraft benötigt, während diese Kraft dort, wo sich die Hülsenarme treffen, verhältnismäßig hoch ist. Der Schlitz öffnet sich somit zunächst an den distalen Enden der Hülsenarme und die Öffnung setzt sich in Richtung proximal fort, sodass das Implantat im Zentrum später freigesetzt wird. Dieses Zentrum entspricht der Stelle, die typischerweise vor dem Aneurysmahals platziert wird.

Alternativ kann die Öffnungszone eine in distaler Richtung weisende Schwächungszone aufweisen, wobei die Schwächungszone so beschaffen ist, dass die zur Öffnung benötigte Kraft von distal nach proximal zunimmt. Die Schwächungszone kann beispielsweise über eine Perforation erreicht werden, bei der die Stärke der Stege zwischen den Öffnungen von distal nach proximal und/oder die Größe der Öffnungen von proximal nach distal zunimmt. Eine andere Möglichkeit besteht darin, im Bereich der Schwächungszone eine Materialverdünnung oder auch eine Variation des Materials vorzusehen, so dass die Schwächungszone an den distalen Enden der Hülsenarme zuerst aufreißt, wenn die Hülse nach proximal zurückgezogen wird, und die Öffnung der Schwächungszone sich nach proximal, d. h. zur Verzweigungsstelle der Hülse fortpflanzt. Die Schwächungszone ist somit so beschaffen, dass sie beim Zurückziehen der Hülse von den distalen Enden der Hülsenarme zur Verbindungsstelle der Hülsenarme aufreißt. Auch auf diese Weise wird eine sequentielle Freisetzung der distalen Implantatarme sichergestellt, bei der zuerst die weiter distal liegenden Bereiche freigesetzt werden und expandieren und erst geringfügig später die weiter proximal liegenden Bereiche.

Ein weiteres Einführsystem, mit dem das erfindungsgemäße Implantat vor dem Aneurysma platziert werden kann, weist zwei Hülsen auf, die zur Aufnahme jeweils eines distalen Implantatarms konfiguriert sind, wobei die beiden Hülsen jeweils über einen distalen Hülsenabschnitt verfügen und die distalen Hülsenabschnitte jeweils eine in Längsrichtung verlaufende Öffnungszone aufweisen, wobei sich proximal an die distalen Hülsenabschnitte jeweils ein proximaler Abschnitt anschließt, über den sich die Hülsen in proximaler Richtung zurückziehen lassen, sodass sich die Öffnungszonen öffnen und die distalen Implantatarme jeweils durch die Öffnungszonen treten und in den abzweigenden Blutgefäßen freigesetzt werden. Darüber hinaus ist eine Stammhülse vorgesehen, die für die Aufnahme des proximalen Implantatarms vorgesehen ist, der im Stammblutgefäß platziert wird. Die Stammhülse lässt sich zur Freisetzung des proximalen Implantatarms unabhängig von den Hülsen für die distalen Implantatarme in proximaler Richtung zurückziehen bzw. die Stammhülse kann festgehalten werden, um auch den proximalen Implantatarm durch Vorschub in Richtung distal freizusetzen. Die Stammhülse muss sich somit in Richtung proximal fortsetzen, wobei der proximale Bereich nicht unbedingt hülsenförmig sein muss, sondern z. B. auch aus einem Draht o. ä. bestehen kann.

Bei diesem Einführsystem ist eine Hülse für jeden der beiden distalen Implantatarme vorgesehen, der in einem der vom Stammblutgefäß abzweigenden Blutgefäße platziert werden sollen. Die Hülse schränkt die Implantatarme ein und hindert sie an einer radialen Expansion. Zumindest im distalen Bereich weisen beide Hülsen eine in Längsrichtung verlaufende Öffnungszone auf, durch die die distalen Implantatarme treten können und freigesetzt werden, wenn die Hülsen in proximaler Richtung zurückgezogen werden. Alternativ kann die Freisetzung auch dadurch erfolgen, dass die Hülsen mit Hilfe der proximalen Abschnitte festgehalten, das Implantat mit den beiden distalen Implantatarmen hingegen in distaler Richtung vorgeschoben wird. Wichtig ist für die Freisetzung die Relativbewegung zwischen Hülsen und Implantat, wobei die Relativbewegung der Hülsen in Richtung proximal, die Relativbewegung des Implantats hingegen in Richtung distal wirkt. In diesem Zusammenhang kann eine Vorschubeinrichtung verwendet werden, um eine Kraft auf das Implantat in proximaler Richtung auszuüben, entweder um dieses zu fixieren, während die Hülsen nach proximal gezogen werden, oder um das Implantat nach distal vorzuschieben.

Unter einer Öffnungszone wird in diesem Zusammenhang ein Bereich der distalen Hülsenabschnitte verstanden, der in Längsrichtung vom distalen Ende der distalen Hülsenabschnitte aus in Richtung proximal verläuft, typischerweise bis zum Beginn der proximalen Abschnitte. Die Öffnungszonen sollten dabei vorzugsweise in Richtung distal weisen, um den Austritt der distalen Implantatarme zu erleichtern. Die Öffnungszonen müssen sich soweit in Richtung proximal erstrecken, dass ein zumindest ausreichend langer Abschnitt des Implantats von den Hülsen aufgenommen werden kann und während der Einführung das Implantat sicher vom Einführsystem gehalten wird. An den proximalen Enden der distalen Hülsenabschnitte hingegen treten die Implantatarme aus den Hülsen heraus, während die Hülsen sich in proximaler Richtung weiter erstreckende proximale Abschnitte aufweisen.

Bei den Öffnungszonen kann es sich insbesondere um Längsschlitze handeln, durch die distalen Implantatarme treten können, wenn die Hülsen in proximaler Richtung zurückgezogen werden. Die in Längsrichtung verlaufenden Kanten der Längsschlitze können aneinanderstoßen oder sich in gewissem Maße überlappen, wobei jeweils sichergestellt werden muss, dass ein Durchtritt der Implantatarme möglich bleibt. Längsrichtung bedeutet in diesem Zusammenhang, dass die Öffnungszone vom distalen Ende der distalen Hülsenabschnitte aus in Richtung proximal verlaufen muss, wobei auch ein Verlauf mit einem gewissen radialen Anteil als "in Längsrichtung" verstanden wird, beispielweise ein helikaler Verlauf.

Möglich ist statt des Vorsehens eines Längsschlitzes auch das Vorsehen einer sonstigen Öffnungszone, bei der der Längsschlitz erst während des Freisetzungsvorgangs entsteht. Beispielsweise kann es sich um eine Schwächungszone handeln, bei der eine Perforation vorgesehen ist, die sich beim Zurückziehen der Hülsenabschnitte öffnet. Ebenso möglich ist eine anderweitige Schwächungszone, die auf einer Materialverdünnung basiert.

Sinnvoll ist auch ein Vorsehen der Öffnungszone in der Weise, dass die freizusetzenden distalen Implantatarme von distal nach proximal freigesetzt werden, d. h. zunächst die beiden distalen Enden des Implantats, bis schließlich auch die am weitesten proximal liegenden Bereiche der distalen Implantatarme, die sich innerhalb der Hülse befinden, aus den distalen Hülsenabschnitten austreten. Dies kann z. B. dadurch erreicht werden, dass die in proximaler Richtung auszuübende Kraft für die Öffnung der Öffnungszone von distal nach proximal zunimmt. So kann etwa die Widerstandskraft einer Schwächungszone von distal nach proximal zunehmen, indem die Materialstärke ansteigt, unterschiedliche Materialien verwendet werden oder eine Perforation mit hinsichtlich der Stärke in Richtung proximal anwachsenden Zwischensegmenten und/oder in Richtung distal anwachsenden Öffnungen vorgesehen wird. Im Falle eines Längsschlitzes kann die Überlappung der Kanten des Längsschlitzes von distal nach proximal zunehmen, was ebenfalls zur Folge hat, dass die distalen Enden des Implantats zuerst freigesetzt werden. Mit Hilfe des beschriebenen Einführsystems können die einzelnen Implantatarme durch Rückzug der Hülsen bzw. der Stammhülse in proximaler Richtung separat freigesetzt werden. Zumindest im Bereich der distalen Hülsenabschnitte sind die Hülsen sinnvollerweise aus einem flexiblen Schlauchmaterial gefertigt, sodass sich die Öffnungszonen gut öffnen lassen und das Implantat austreten kann. Auch die Stammhülse kann aus einem flexiblen Schlauchmaterial gefertigt sein.

Ein weiteres verwendbares Einführsystem weist zwei distale Schaftabschnitte auf, die durch die beiden distalen Implantatarme verlaufen, sowie einen proximalen Schaftabschnitt, der durch den proximalen Implantatarm verläuft. Die drei Schaftabschnitte sind angrenzend an die Verzweigungsstelle des Implantats miteinander verbunden. Über den proximalen Schaftabschnitt lassen sich auch die distalen Schaftabschnitte in proximaler Richtung zurückziehen. Die distalen Implantatarme sind an den distalen Schaftabschnitten lösbar festgelegt, sodass die distalen Implantatarme nach Lösen der Festlegung ihre expandierte Struktur einnehmen und in den abzweigenden Blutgefäßen freigesetzt werden.

Bei diesem Einführsystem wird das Implantat somit nicht innerhalb einer Hülse an die gewünschte Stelle gebracht, sondern auf den Schaftabschnitten des Einführsystems festgelegt. Mit anderen Worten wird das Implantat auf dem Einführsystem an die gewünschte Stelle gebracht. Sobald die korrekte Platzierung erfolgt ist, wird die Festlegung des Implantats an den Schaftabschnitten gelöst. Das Implantat weitet sich daraufhin auf und legt sich an die Gefäßinnenwandung an, wobei die beiden distalen Implantatarme sich an die Gefäßinnenwandlung der beiden abzweigenden Blutgefäße anlegen.

Die Festlegungsstellen zwischen den distalen Implantatarmen und den distalen Schaftabschnitten sind sinnvollerweise zumindest an den distalen Enden des Implantats angeordnet. Sobald die Festlegungsstellen an den distalen Enden gelöst werden, beginnt die Expansion des Implantats. Möglich sind auch weitere Festlegungsstellen weiter proximal, diese sind jedoch nicht unbedingt erforderlich, weil die Freisetzung weiter proximal auch über einen das Implantat einschließlich des Einführsystems umgebenden Mikrokatheter gesteuert werden kann.

Die Schaftabschnitte können insbesondere aus einem flexiblen Schlauchmaterial aufgebaut sein, was mit dem Vorteil verbunden ist, dass sich das Implantat gut an das Schlauchmaterial anlegt und ein zusätzlicher Reibschluss zwischen Implantat und Schlauchmaterial hergestellt wird. Grundsätzlich denkbar ist jedoch auch beispielsweise ein metallischer Schaft, auf dem das Implantat aufgebracht und festgelegt ist.

Die Schaftabschnitte weisen zweckmäßigerweise einen inneren Hohlraum auf, weil dies die Durchführung eines oder mehrerer Führungsdrähte ermöglicht, über die das Einführsystem an die gewünschte Stelle gebracht werden kann. Insbesondere können auch mehrere Führungsdrähte verwendet werden, von denen beispielsweise einer in das erste, der andere in das zweite abzweigende Gefäß geführt wird, um das Einführsystem an die gewünschte Stelle vorschieben zu können.

Die Festlegungsstellen können Verbindungsstellen sein, an denen eine chemisch, thermisch, elektrolytisch oder mechanisch lösbare Verbindung vorliegt. Das Lösen der Verbindungsstellen kann entsprechend über die Einstellung entsprechender Parameter gesteuert werden.

Unter chemisch, thermisch oder elektrolytisch lösbaren Verbindungsstellen werden Verbindungsstellen verstanden, die sich durch chemische oder thermische Einwirkung oder elektrolytisch durch Anlegen einer elektrischen Spannung zumindest so weitgehend auflösen lassen, dass sich der jeweilige Arm des Implantats vom Schaftabschnitt löst.

Bei den Verbindungsstellen kann es sich um Klebeverbindungsstellen handeln, wobei bevorzugt ein Polymerklebstoff verwendet wird. Die Ablösung des Implantats erfolgt in diesem Fall chemisch, nämlich typischerweise durch Applikation eines den Klebstoff zumindest teilweise auflösenden Lösungsmittels. Als Lösungsmittel kann z. B. DMSO (Dimethylsulfoxid) verwendet werden. Unter einer chemisch lösbaren Verbindungsstelle wird auch eine Verbindungsstelle verstanden, die sich allein durch Einwirkung des umgebenden Blutes löst.

Eine weitere Möglichkeit besteht in der elektrolytischen Ablösung der Verbindungsstellen. Die zumindest partielle Auflösung der Verbindungsstelle erfolgt in diesem Fall durch Anlegen einer elektrischen Spannung. Die elektrolytische Ablösung von Implantaten ist aus dem Stand der Technik hinlänglich bekannt. Als Materialien für die elektrolytisch aufzulösende Verbindungsstelle eignen sich beispielsweise Edelstahl, Magnesium, Magnesiumlegierungen oder Cobalt-Chrom-Legierungen. Eine besonders bevorzugte Magnesiumlegierung ist Resoloy^{®}, welche von der Firma MeKo aus Sarstedt/Deutschland entwickelt wurde (vgl. WO 2013/024125 A1). Es handelt sich um eine Legierung aus Magnesium und u. a. Lanthanoiden, insbesondere Dysprosium. Vorteilhaft bei der Verwendung von Magnesium und Magnesiumlegierungen ist auch, dass ein Verbleib von Magnesiumresten im Körper physiologisch unproblematisch ist.

Im Falle einer thermisch lösbaren Verbindungsstelle kann zur Ablösung des Implantats eine Wärmequelle an die zu lösenden Verbindungsstellen gebracht werden.

Eine weitere Möglichkeit, die für die Platzierung in den abzweigenden Blutgefäßen vorgesehenen Implantatarme an den Schaftabschnitten festzulegen, besteht darin, auf die distalen Enden der distalen Implantatarme Kappen zu setzen, die eine radiale Aufweitung der Implantatarme verhindern. Diese Kappen sind in distaler Richtung von den distalen Enden entfernbar, insbesondere abschiebbar, um auf diese Weise eine Freisetzung des Implantats zu erreichen.

Die Kappen können insbesondere zylindrisch ausgebildet sein, wobei in der Regel das distale Ende der Kappen weitgehend geschlossen ist, abgesehen von einer kleinen Öffnung zur Durchführung eines Drahts wie weiter unten beschrieben. Der Innendurchmesser der Kappen muss so beschaffen sein, dass eine Aufweitung und Freisetzung der distalen Implantatarme bei aufgesetzter Kappe verhindert wird.

Das Ablösen der Kappen kann insbesondere dadurch herbeigeführt werden, dass Schubdrähte zumindest temporär durch die inneren Hohlräume der distalen Schaftabschnitte geführt werden. Diese Schubdrähte sind so gestaltet, dass der Vorschub der Schubdrähte in distaler Richtung ein Abschieben der Kappen von den distalen Enden des Implantats herbeiführt. Hierfür können die Schubdrähte beispielsweise Verdickungen aufweisen, die am oder nahe des distalen Endes angebracht sind.

Besonders bevorzugt ist das Vorsehen von Schubdrähten, die durch hierfür vorgesehene Öffnungen in den Kappen verlaufen, wobei die Schubdrähte distal und proximal der Öffnungen Verdickungen aufweisen und die weiter proximal gelegene Verdickung eines Schubdrahts dafür sorgt, dass sich die Verdickung beim Vorschub in Richtung distal an das Innere der Kappe anlegt und diese bei weiterer Kraftausübung vom Implantat abschiebt, während die distalen Verdickungen dafür sorgen, dass die Schubdrähte in ihren Positionen gehalten werden und nicht aus den Öffnungen in den Kappen herausrutschen. Die Verdickungen sollten einen Durchmesser aufweisen, der größer ist als der Durchmesser der für die Schubdrähte vorgesehenen Öffnungen in den Kappen, da anderenfalls ein Lösen der Schubdrähte von den Kappen möglich wäre. Insbesondere können die Verdickungen kugelförmig ausgebildet sein.

Bei Verwendung eines der beschriebenen oder auch eines beliebigen anderen Einführsystems können Implantat und Einführsystem über einen Mikrokatheter an die Zielposition gebracht werden.

Neben dem Implantat selbst betrifft die Erfindung auch die Verwendung des Implantats zur Behandlung arteriovenöser Fehlbildungen, insbesondere (Bifurkations)aneurysmen sowie die Kombination des Implantats mit einem Einführsystem und ggf. einem Mikrokatheter. Sämtliche Ausführungen, die zum Implantat selbst gemacht wurden, gelten in entsprechender Weise auch für die Verwendung des Implantats und ein Verfahren zur Anwendung des Implantats.

Die Erfindung wird anhand der Figuren beispielhaft näher erläutert. Es ist darauf hinzuweisen, dass die Figuren bevorzugte Ausführungsvarianten der Erfindung zeigen, die Erfindung ist jedoch nicht darauf beschränkt. Insbesondere umfasst die Erfindung, soweit es technisch sinnvoll ist, beliebige Kombinationen der technischen Merkmale, die in den Ansprüchen aufgeführt oder in der Beschreibung als erfindungsrelevant beschrieben sind.

Es zeigen:
- Fig. 1: Einen ersten Geflechtabschnitt gemäß der ersten Ausführungsform;
- Fig. 2: einen zweiten Geflechtabschnitt gemäß der ersten Ausführungsform;
- Fig. 3: das aus erstem und zweitem Geflechtabschnitt zusammengefügte Implantat gemäß erster Ausführungsform;
- Fig. 4: eine Geflechtstruktur gemäß zweiter Ausführungsform in der Draufsicht;
- Fig. 5: eine Geflechtstruktur gemäß zweiter Ausführungsform in der Seitenansicht; und
- Fig. 6: das aus der Geflechtstruktur geformte Implantat gemäß zweiter Ausführungsform.

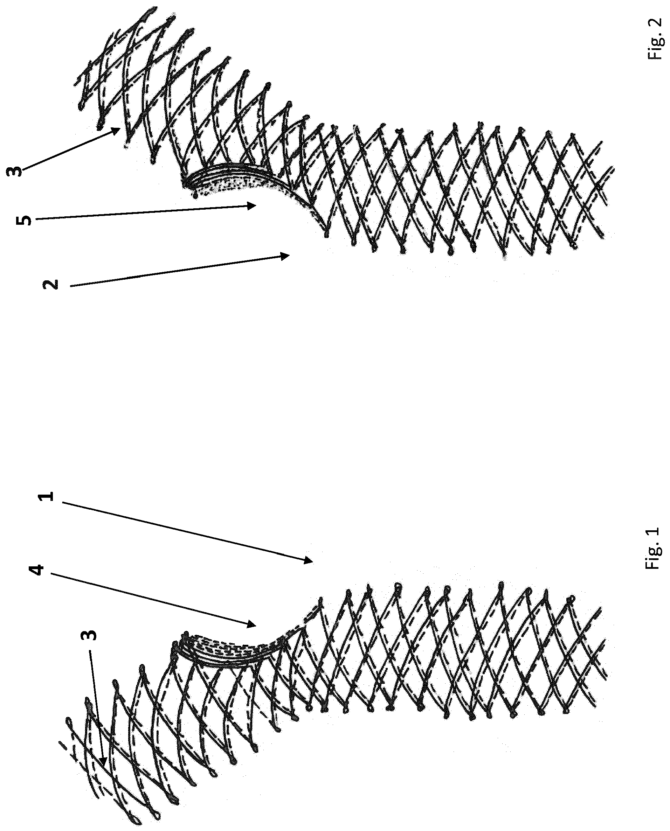

In den Figuren 1 bis 3 ist die erste Ausführungsform der Erfindung dargestellt. In Figur 1 wird der erste Geflechtabschnitt 1 gemäß der ersten Ausführungsform der Erfindung dargestellt. Dieser ist aus einer Vielzahl von Drähten 3 aufgebaut, die miteinander verflochten sind. Drähte 3 im Vordergrund sind mit einer durchgezogenen Linie dargestellt, Drähte 3 im Hintergrund mit einer gestrichelten Linie. Der Geflechtabschnitt 1 bildet im expandierten Zustand im Wesentlichen eine röhrenförmige Struktur, die eine Öffnung 4 in der Wandung mit einer Größe aufweist, dass der andere Geflechtabschnitt 2 durch die Öffnung hindurchgeführt werden kann.

Figur 2 entspricht Figur 1, zeigt jedoch den zweiten Geflechtabschnitt 2, der im Wesentlichen identisch aufgebaut ist, d. h. ebenfalls aus miteinander verflochtenen Drähten 3. Auch dieser Geflechtabschnitt 2 weist eine Öffnung mit einer Größe auf, dass der andere Geflechtabschnitt 1 hindurchgeführt werden kann.

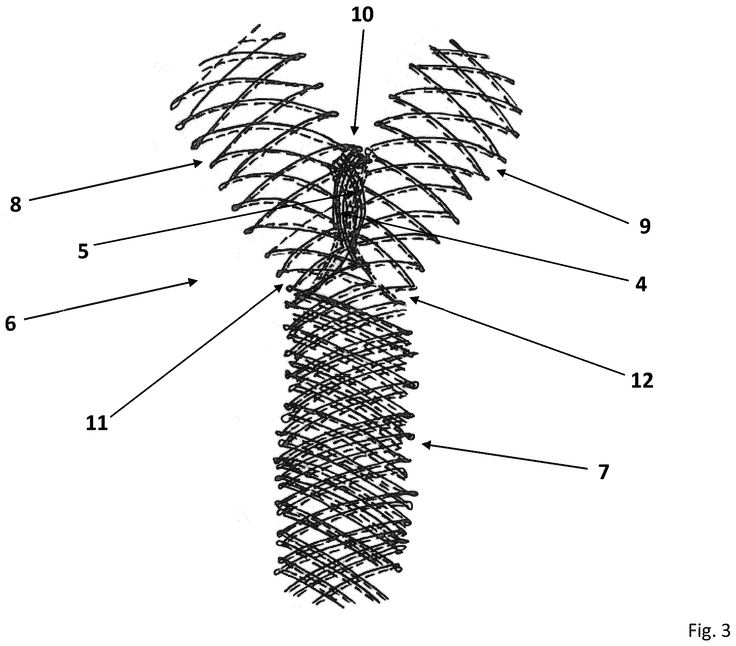

In Figur 3 ist das aus dem ersten und dem zweiten Geflechtabschnitt 1, 2 zusammengefügte Implantat 6 zu sehen. Das Implantat 6 hat eine Y-Form, wobei die beiden Öffnungen 4, 5 in den Geflechtabschnitten 1, 2 aufeinander zuweisen, sodass das Blut zwischen den Armen 7, 8, 9 problemlos fließen kann. Einer der Arme 7 ist doppellagig, weil in diesem Bereich die beiden Geflechtabschnitte 1, 2 übereinander liegen. Die beiden anderen Arme 8, 9 sind jeweils einlagig, weil hier nur der erste bzw. der zweite Geflechtabschnitt 1, 2 vorliegen. Entsprechend ist die Oberflächenabdeckung im doppellagigen Arm 7 größer als in den beiden einlagigen Armen 8, 9.

Gemäß der Standardplatzierung wird das Implantat 6 so eingebracht, dass der Bereich 10 vor dem Aneurysma bzw. im Aneurysmahals zu liegen kommt. Entsprechend ist der doppellagige Arm 7 im Stammblutgefäß verankert, während die beiden einlagigen Arme 8, 9 in die abzweigenden Blutgefäße hineinragen. Über die zueinander zeigenden Öffnungen 5, 6 in den Geflechtabschnitten 1, 2 kann das Blut problemlos vom Stammblutgefäß in die beiden abzweigenden Blutgefäße fließen. Auf der anderen Seite wird das Aneurysma weitgehend vom Blutfluss abgeschnitten, da an der Position 10 eine ausreichend hohe Geflechtdichte vorliegt.

Alternativ kann das Implantat 6 auch so platziert werden, dass entweder der Bereich 11 oder der Bereich 12 vor dem Aneurysma zu liegen kommt. In diesem Fall befindet sich der doppellagige Arm 7 in einem der abzweigenden Blutgefäße, während einer der beiden einlagigen Arme 8, 9 im Stammblutgefäß positioniert wird. Dies kann insofern vorteilhaft sein, als in den Bereichen 11 und 12 in der Regel eine noch höhere Geflechtdichte vorliegt als im Bereich 10.

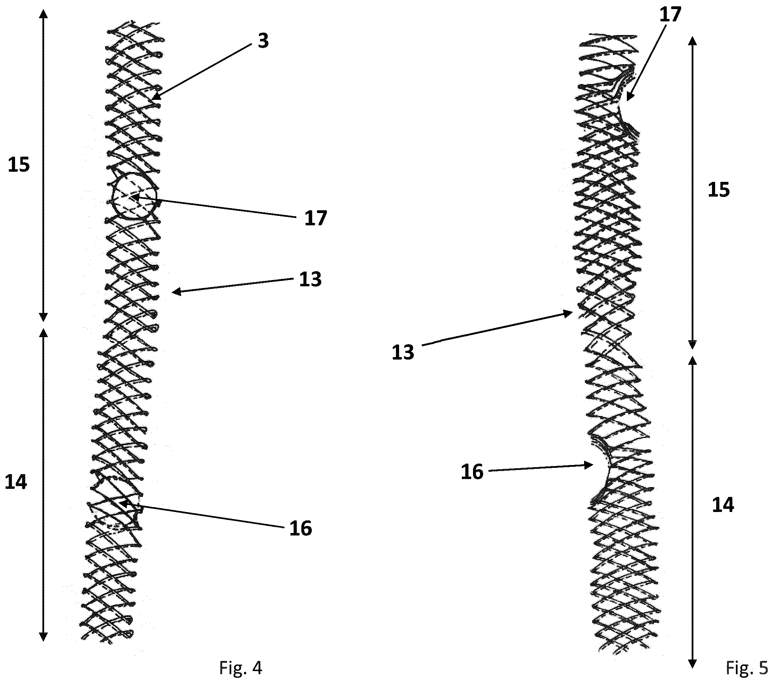

In den Figuren 4 bis 6 wird die zweite Ausführungsform der Erfindung verdeutlicht. Figur 4 zeigt die Geflechtstruktur 13, die einen ersten und einen zweiten Geflechtabschnitt 14, 15 aufweist. Anders als bei der ersten Ausführungsform sind beide Geflechtabschnitte 14, 15 jedoch Bestandteil einer einzelnen Geflechtstruktur 13 und lediglich in Längsrichtung hintereinander angeordnet. In beiden Geflechtabschnitten 14, 15 ist jeweils eine Öffnung 16, 17 zu erkennen, wobei die Öffnung 17 in der hier gewählten Darstellung nach oben, die Öffnung 16 in der hier gewählten Darstellung nach unten zeigt. Die Öffnungen 16, 17 befinden sich somit auf entgegengesetzten Seiten. Auch die Geflechtstruktur 13 ist aus miteinander verflochtenen Drähten 3 gebildet, wobei wiederum im Vordergrund befindliche Drähte 3 mit einer durchgehenden Linie, im Hintergrund liegende Drähte 3 mit einer gestrichelten Linie dargestellt werden.

Figur 5 entspricht der Darstellung aus Figur 4, jedoch in der Seitenansicht. Hier erkennt man gut die entgegengesetzte Anbringung der Öffnungen 16, 17.

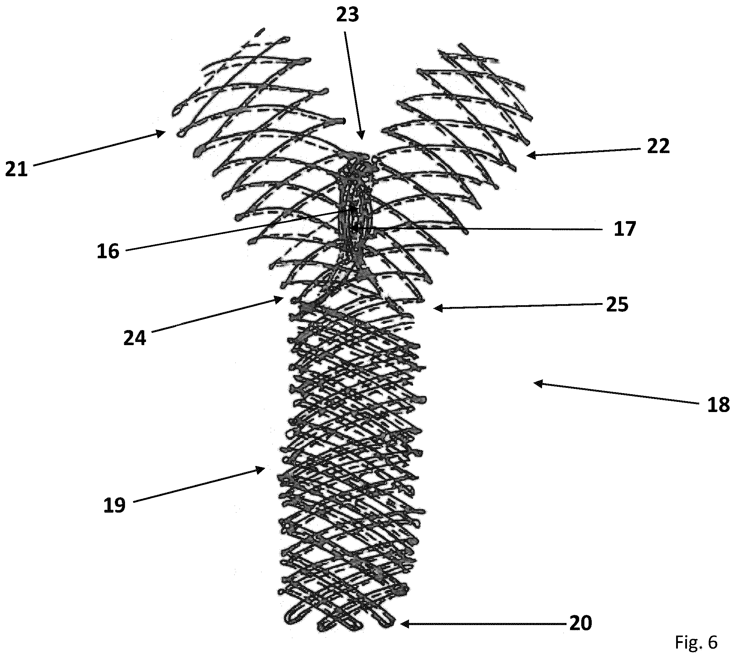

In Figur 6 schließlich ist das Implantat 18 gemäß der zweiten Ausführungsform im Ganzen dargestellt, nachdem es in die entsprechende Form gebracht wurde. Hierzu wurde der erste Geflechtabschnitt 14 nach innen gestülpt und durch die Geflechtstruktur 13 durchgeführt, um ihn an der Öffnung 17 im zweiten Geflechtabschnitt 15 wieder austreten zu lassen. Man erhält somit insgesamt wiederum ein Y-förmiges Implantat 18 mit drei Armen 19, 21, 22, von denen ein Arm 19 doppellagig ist, die beiden anderen Arme 21, 22 hingegen einlagig. Anders als bei der ersten Ausführungsform weist allerdings das äußere Ende 20 des doppellagigen Arms 19 keine freien Drahtenden auf, sondern die äußere Lage ist mit der inneren Lage verbunden, was sich aus der beschriebenen Herstellungsweise ergibt. Das Fehlen von freien Drahtenden ist insofern vorteilhaft, als es das Implantat 18 an dieser Stelle weniger traumatisch macht, d. h. die Gefahr von Verletzungen der Gefäßwandung ist reduziert.

Auch bei der zweiten Ausführungsform zeigen die beiden Öffnungen 16, 17 zueinander, sodass der Blutfluss durch das Stammblutgefäß und die beiden abzweigenden Blutgefäße ungehindert ist. Auf der anderen Seite jedoch wird das Aneurysma, vor dem das Implantat 18 implantiert wird, wirkungsvoll vom Blutfluss abgeschnitten, weil das Implantat 18 an der Position, an der es vor dem Aneurysmahals zu liegen kommt, eine ausreichend hohe Oberflächendichte aufweist. In der Standardpositionierung handelt es sich hierbei um den Bereich 23, auch bei dieser Ausführungsform ist es jedoch alternativ möglich, die Bereiche 24 oder 25 vor dem Aneurysma zu platzieren, um unmittelbar vor dem Aneurysmahals eine noch höhere Geflechtdichte zu erreichen. Während bei der Standardpositionierung der doppellagige Arm 19 im Stammblutgefäß platziert wird, befindet sich der doppellagige Arm 19 bei den beiden alternativen Positionierungen in einem der abzweigenden Gefäße. Einer der beiden einlagigen Arme 21, 22 muss entsprechend im Stammblutgefäß platziert werden.

## Patentansprüche

1. Implantat zur Beeinflussung des Blutflusses im Bereich von Aneurysmen, die an Gefäßverzweigungen lokalisiert sind, wobei das Implantat (6, 18) in einem expandierten Zustand, in dem es im Blutgefäß implantiert wird, und in einem kontrahierten Zustand vorliegt, in dem es durch das Blutgefäß bewegbar ist, wobei das Implantat (6, 18) einen ersten (1, 14) und einen zweiten Geflechtabschnitt (2, 15) aufweist, welche im expandierten Zustand röhrenförmig und deren Wandungen aus einzelnen miteinander verflochtenen Drähten (3) aufgebaut sind, wobei der erste (1, 14) und der zweite Geflechtabschnitt (2, 15) jeweils eine Öffnung (4, 5, 16, 17) in der Wandung aufweisen und zumindest die Größe der Öffnung (5, 17) im zweiten Geflechtabschnitt (2, 15) ausreichend ist für die Hindurchführung des ersten Geflechtabschnitts (1, 14), wobei der erste Geflechtabschnitt (1, 14) so durch die Öffnung (5, 17) in der Wandung des zweiten Geflechtabschnitts (2, 15) geführt ist, dass die Öffnung (4, 16) in der Wandung des ersten Geflechtabschnitts (1, 14) zur Öffnung (5, 17) in der Wandung des zweiten Geflechtabschnitts (2, 15) weist,
**dadurch gekennzeichnet,**
**dass** die Öffnungen (4, 5, 16, 17) in den Wandungen dadurch erzeugt sind, dass die die Geflechtabschnitte (1, 2, 14, 15) ausbildenden Drähte (3) an den Positionen der Öffnungen (4, 5, 16, 17) radial verdrängt werden, und das Implantat (6, 18) vor der Einführung in das Blutgefäß zusammengefügt wird und als Einheit an die Zielposition bringbar ist.

2. Implantat nach Anspruch 1, **dadurch gekennzeichnet, dass** die Zahl der Drähte (3), die die jeweiligen Geflechtabschnitte (1, 2, 14, 15) ausbilden, 24 bis 96, insbesondere 36 bis 64 beträgt.

3. Implantat nach Anspruch 1 oder 2, **dadurch gekennzeichnet, dass** sich im expandierten Zustand der erste (1) und der zweite Geflechtabschnitt (2) in dem Segment (7), in dem die beiden Geflechtabschnitte (1, 2) gemeinsam verlaufen, überlappen, in der Weise, dass der innenliegende Geflechtabschnitt (1) über den außen liegenden Geflechtabschnitt (2) hinausragt.

4. Implantat nach Anspruch 1 oder 2, **dadurch gekennzeichnet, dass** der erste (14) und der zweite Geflechtabschnitt (15) in dem Segment, in dem die beiden Geflechtabschnitte (14, 15) gemeinsam verlaufen, am äußeren Ende (20) des Segments (19) miteinander verbunden sind.

5. Implantat nach einem der Ansprüche 1 bis 4, **dadurch gekennzeichnet, dass** der erste (1, 14) und der zweite Geflechtabschnitt (2, 15) im Bereich ihrer Öffnungen (4, 5, 16, 17) aneinander befestigt sind.

6. Implantat nach Anspruch 5, **dadurch gekennzeichnet, dass** die Befestigung im Bereich der Öffnungen (4, 5, 16, 17) durch Vernähen und/oder um die Drähte (3) geführte Schlaufen erfolgt.

7. Implantat nach einem der Ansprüche 1 bis 6, **dadurch gekennzeichnet, dass** die Öffnungen (4, 5, 16, 17) in den Wandungen des ersten (1, 14) und/oder zweiten Geflechtabschnitts (2, 15) in Bezug auf die Längserstreckung ungefähr mittig angeordnet sind.

8. Implantat nach einem der Ansprüche 1 bis 7, **dadurch gekennzeichnet, dass** die Drähte (3) zumindest teilweise aus einem Material mit Formgedächtniseigenschaften hergestellt sind.

9. Implantat nach Anspruch 8, **dadurch gekennzeichnet, dass** das Material mit Formgedächtniseigenschaften eine Nickel-Titan-Legierung ist.

10. Implantat nach Anspruch 8 oder 9, **dadurch gekennzeichnet, dass** die Drähte (3) einen Kern aus einem röntgensichtbaren Material und einen Mantel aus einem Material mit Formgedächtniseigenschaften aufweisen.

11. Implantat nach einem der Ansprüche 1 bis 10, **dadurch gekennzeichnet, dass** das Implantat (6, 18) röntgensichtbare Marker aufweist.

12. Implantat nach einem der Ansprüche 1 bis 11, **dadurch gekennzeichnet, dass** das Implantat (6, 18) über eine oder mehrere die Geflechtabschnitte (1, 2, 14, 15) zumindest teilweise abdeckende Membranen verfügt.

13. Implantat nach Anspruch 12, **dadurch gekennzeichnet, dass** die Membran eine antithrombogene oder eine die Endothelbildung fördernde Wirkung aufweist.

## Claims

1. Implant for influencing the blood flow in the region of aneurysms that are located at vascular branches, wherein the implant (6, 18) is present in an expanded state, in which it is implanted in the blood vessel, and in a contracted state, in which it can be moved through the blood vessel, wherein the implant (6, 18) has a first (1, 14) and a second braided portion (2, 15) that are tubular in the expanded state and the walls of which are constructed from individual interwoven wires (3), wherein the first (1, 14) and the second braided portion (2, 15) each have an opening (4, 5, 16, 17) in the wall and at least the size of the opening (5, 17) in the second braided portion (2, 15) is sufficient for the first braided portion (1, 14) to be guided through, wherein the first braided portion (1, 14) is guided through the opening (5, 17) in the wall of the second braided portion (2, 15) such that the opening (4, 16) in the wall of the first braided portion (1, 14) points towards the opening (5, 17) in the wall of the second braided portion (2, 15),
**characterised in that**
the openings (4, 5, 16, 17) in the walls are made such that the wires (3) forming the braided portions (1, 2, 14, 15) are radially displaced at the positions of the openings (4, 5, 16, 17), and the implant (6, 18) is assembled before insertion into the blood vessel and can be brought to the target position as a unit.

2. Implant according to claim 1, **characterised in that** the number of wires (3) that form the respective braided portion (1, 2, 14, 15) is 24 to 96, in particular 36 to 64.

3. Implant according to claim 1 or 2, **characterised in that**, in the expanded state, the first (1) and the second braided portion (2) overlap in the segment (7) in which the two braided portions (1, 2) run together such that the inner braided portion (1) projects beyond the outer braided portion (2).

4. Implant according to claim 1 or 2, **characterised in that** the first (14) and the second braided portion (15) are attached to one another at the outer end (20) of the segment (19) in the segment in which the two braided portions (14, 15) jointly extend.

5. Implant according to any one of claims 1 to 4, **characterised in that** the first (1, 14) and the second braided portion (2, 15) are fastened to each other in the region of their openings (4, 5, 16, 17).

6. Implant according to claim 5, **characterised in that** the fastening in the region of the openings (4, 5, 16, 17) is carried out by sewing and/or loops guided around the wires (3).

7. Implant according to any one of claims 1 to 6, **characterised in that** the openings (4, 5, 16, 17) in the walls of the first (1, 14) and/or second braided portion (2, 15) are arranged approximately centrally with respect to the longitudinal extent.

8. Implant according to any one of claims 1 to 7, **characterised in that** the wires (3) are at least partially made of a material with shape memory properties.

9. Implant according to claim 8, **characterised in that** the material with shape memory properties is a nickel-titanium alloy.

10. Implant according to claim 8 or 9, **characterised in that** the wires (3) have a core made of a radiopaque material and a sheath made of a material with shape memory properties.

11. Implant according to any one of claims 1 to 10, **characterised in that** the implant (6, 18) has radiopaque markers.

12. Implant according to any one of claims 1 to 11, **characterised in that** the implant (6, 18) has one or more membranes at least partially covering the braided portions (1, 2, 14, 15).

13. Implant according to claim 12, **characterised in that** the membrane has an antithrombogenic or endothelium-promoting effect.

## Revendications

1. Implant destiné à influencer le flux sanguin dans la zone d'anévrismes localisés au niveau de ramifications vasculaires, l'implant (6, 18) se présentant dans un état expansé dans lequel il est implanté dans le vaisseau sanguin et dans un état contracté dans lequel il peut se déplacer à travers le vaisseau sanguin, l'implant (6, 18) présentant une première (1, 14) et une deuxième section de treillis (2, 15) qui, à l'état expansé, sont tubulaires et dont les parois sont formées de fils individuels entrelacés (3), la première (1, 14) et la deuxième section de treillis (2, 15) présentant chacune une ouverture (4, 5, 16, 17) dans la paroi et au moins la taille de l'ouverture (5, 17) dans la deuxième section de treillis (2, 15) étant suffisante pour le passage de la première section de treillis (1, 14), la première section de treillis (1, 14) étant guidée à travers l'ouverture (5, 17) dans la paroi de la deuxième section de treillis (2, 15) de telle sorte que l'ouverture (4, 16) dans la paroi de la première section de treillis (1, 14) est orientée vers l'ouverture (5, 17) dans la paroi de la deuxième section de treillis (2, 15),
**caractérisé en ce que**
les ouvertures (4, 5, 16, 17) dans les parois sont produites par le fait que les fils (3) réalisant les sections de treillis (1, 2, 14, 15) sont repoussés radialement aux positions des ouvertures (4, 5, 16, 17), et l'implant (6, 18) est assemblé avant l'introduction dans le vaisseau sanguin et peut être amené en tant qu'unité à la position cible.

2. Implant selon la revendication 1, **caractérisé en ce que** le nombre de fils (3) réalisant les sections de treillis respectives (1, 2, 14, 15) est de 24 à 96, notamment de 36 à 64.

3. Implant selon la revendication 1 ou 2, **caractérisé en ce qu'**à l'état expansé, la première (1) et la deuxième (2) section de treillis dans le segment (7) dans lequel les deux sections de treillis (1, 2) s'étendent ensemble, se chevauchent de telle manière que la section de treillis intérieure (1) dépasse de la section de treillis extérieure (2).

4. Implant selon la revendication 1 ou 2, **caractérisé en ce que** la première (14) et la deuxième (15) section de treillis sont reliées entre elles à l'extrémité extérieure (20) du segment (19) dans le segment dans lequel les deux sections de treillis (14, 15) s'étendent ensemble.

5. Implant selon l'une quelconque des revendications 1 à 4, **caractérisé en ce que** la première (1, 14) et la deuxième (2, 15) section de treillis sont fixées l'une à l'autre dans la zone de leurs ouvertures (4, 5, 16, 17).

6. Implant selon la revendication 5, **caractérisé en ce que** la fixation dans la zone des ouvertures (4, 5, 16, 17) est effectuée par couture et/ou par des boucles guidées autour des fils (3).

7. Implant selon l'une quelconque des revendications 1 à 6, **caractérisé en ce que** les ouvertures (4, 5, 16, 17) dans les parois de la première (1, 14) et/ou de la deuxième section de treillis (2, 15) sont agencées approximativement au centre par rapport à l'extension longitudinale.

8. Implant selon l'une quelconque des revendications 1 à 7, **caractérisé en ce que** les fils (3) sont au moins partiellement fabriqués dans un matériau présentant des propriétés de mémoire de forme.

9. Implant selon la revendication 8, **caractérisé en ce que** le matériau présentant des propriétés de mémoire de forme est un alliage de nickel-titane.

10. Implant selon la revendication 8 ou 9, **caractérisé en ce que** les fils (3) présentent un noyau en un matériau visible aux rayons X et une enveloppe en un matériau présentant des propriétés de mémoire de forme.

11. Implant selon l'une quelconque des revendications 1 à 10, **caractérisé en ce que** l'implant (6, 18) présente des marqueurs visibles aux rayons X.

12. Implant selon l'une quelconque des revendications 1 à 11, **caractérisé en ce que** l'implant (6, 18) dispose d'une ou plusieurs membranes recouvrant au moins partiellement les sections de treillis (1, 2, 14, 15).

13. Implant selon la revendication 12, **caractérisé en ce que** la membrane présente un effet antithrombogène ou favorisant la formation d'endothélium.
